(19) Europäisches Patentamt
European Patent Office
Office européen des brevets

(11) Publication number: **0 126 558 A1**

(12) **EUROPEAN PATENT APPLICATION**

(21) Application number: **84302817.6**

(22) Date of filing: **26.04.84**

(51) Int. Cl.³: **C 07 C 129/16**, C 07 D 295/20, A 01 N 47/44, A 61 K 7/22

(30) Priority: **09.05.83 GB 8312661**

(43) Date of publication of application: **28.11.84 Bulletin 84/48**

(84) Designated Contracting States: **AT BE CH DE FR GB IT LI LU NL SE**

(71) Applicant: **IMPERIAL CHEMICAL INDUSTRIES PLC, Imperial Chemical House Millbank, London SW1P 3JF (GB)**

(72) Inventor: **Gunn, Donald Murray, 16 Merlin Park, Dollar Clackmannan Scotland FK14 7BZ (GB)**
Inventor: **Pemberton, Dennis, 9 Eskdale Avenue, Bramhall Stockport Cheshire (GB)**

(74) Representative: **Atkinson, John David et al, Imperial Chemical Industries PLC Legal Department : Patents PO Box 6 Bessemer Road, Welwyn garden City Herts, AL7 1HD (GB)**

(54) **Bisbiguanide derivatives.**

(57) This invention relates to a bisbiguanide of the formula:

$R^1R^1N.C(:NR^5)NH.C(:NH)NH\text{-}X\text{-}NH.C(:NH)NH.C(:NR^6)NR^3R^4$ VIII

or a tautomeric form thereof, wherein each of $R^1$, $R^2$, $R^3$, and $R^4$, which may be the same or different, is hydrogen, an alkyl radical of up to 5 carbon atoms (1-5C) such that $R^1$, $R^2$, $R^3$ and $R^4$ together contain 4 to 12 carbon atoms, or a 3-5C alkenyl radical; or $R^1$, $R^2$ and the nitrogen atom to which they are attached, or $R^3$ and $R^4$ and the nitrogen atom to which they are attached, which may be the same or different, are each a 1-azetidinyl, 1-pyrrolidinyl, piperidino, hexamethyleneimino, heptamethyleneimino, morpholino or 4-(1-8C alkanoyl)-1-piperazinyl radical, each of which may bear 1-3C alkyl substituents; provided that at least one of $R^1$, $R^2$, $R^3$, and $R^4$ is other than hydrogen; $R^5$ and $R^6$ are each hydrogen or a 1-8C alkyl radical; and X is a 7-20C linking group which is a straight chain alkylene radical, or a straight chain alkylene radical bearing one or more 1-3C alkyl substituents, of which any pair or pairs which are attached to different carbon atoms may be joined so as to form, together with the intervening carbon atom or atoms of the alkylene radical, a cycloalkyl radical or radicals; and the acid addition salts thereof; to processes for the manufacture thereof; and to compositions and methods of use thereof.

EP 0 126 558 A1

PH.32712/EP
0126558

# TITLE: BISBIGUANIDE DERIVATIVES

This invention relates to novel bisbiguanide derivatives which possess antibacterial properties.

In United Kingdom patent specification No. 705,838 there are disclosed and claimed bisbiguanides of the general formula:-

$$A\text{-}NH.C(:NH)NH.C(:NH)NH\text{-}(CH_2)_n\text{-}NH.C(:NH)NH.C(:NH)NH\text{-}A \quad I$$

wherein A stands for a phenyl radical which is substituted by alkyl, alkoxy, nitro or halogen, and wherein the two A's may be the same or different, and wherein n is an integer from 3 to 9 inclusive, and wherein the polymethylene chain may be interrupted by oxygen atoms and/or by aromatic nuclei. The compounds are said to be useful as bactericides; for example, those of the formula:-

$$A'\text{-}NH.C(:NH)NH.C(:NH)NH\text{-}(CH_2)_{5\text{-}7}\text{-}NH.C(:NH)NH.C(:NH)NH\text{-}A' \quad II$$

wherein A' stands for a halogen substituted phenyl radical possess very high antibacterial activity when tested _in vitro_ against the organisms _Streptococcus haemolyticus_, _Staphylococcus aureus_, _Bacillus coli_, _Clostridium welchii_ and _Pseudomonas pyocyanea_.

A paper by Rose and Swain, J. Chem. Soc., 1956, 4422-4425, is concerned with bisbiguanides of the above, and other, types. The authors indicated that compounds containing only one biguanide unit were but weakly antibacterial, but that full biological activity

was achieved with compounds containing two biguanide units. They stated that effort was then concentrated on ascertaining the optimum distance which should exist between the two biguanide units, and the most effective types of end groups - whether, for example, antibacterial activity was highest in bisbiguanides in which the terminal groups were aryl, alkyl or heterocyclic. They further stated that the highest antibacterial activity had been found with diaryl compounds of the formula:-

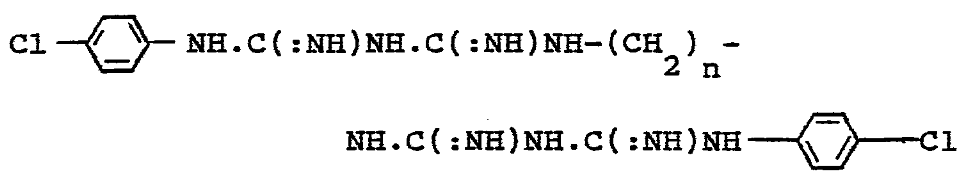

III

and in particular when n stood for 5, 6 or 7. They also stated that analogous alkyl-bisbiguanides of the formula:-

$$RR'N.C(:NH)NH.C(:NH)NH-(CH_2)_6-NH.C(:NH)NH.C(:NH)NRR'$$

IV

wherein R stands for an alkyl radical and R' stands for hydrogen or an alkyl radical, were only fractionally as effective (one-third to one-tenth) as the compounds of the formula III. Specific alkyl-bisbiguanides of the formula IV which are described in the paper are those wherein both R's stand for hydrogen, and a related compound in which both R's stand for a cyclohexyl radical. An essentially similar teaching is to be found in a paper by Davies et al., Brit.J.Pharmacol., 1954, 9, 192-196.

In United Kingdom patent specification No.1,095,902 there is disclosed and claimed a broad group of bisguanides and bisbiguanides which includes <u>inter alia</u> compounds of the formula:-

$$RR'N-[-C(:NH)NH-]_x-A^2-[NH.C(:NH)-]_x-NRR' \qquad V$$

wherein $A^2$ stands for an alkylene radical of 2 to 12 carbon atoms having the valency bonds attached to different carbon atoms, or for a group of the formula:-

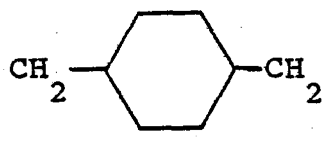

VI

R stands for an alkyl radical of 6 to 16 carbon atoms, R' stands for hydrogen, and x stands for 1 or 2. The bisguanides and bisbiguanides are said to have particular usefulness as plant fungicides and bactericides. On page 6 of the said specification No. 1,095,902 data are presented which illustrate the high degree of bacteriostatic, bactericidal, fungistatic and fungicidal activity possessed by the "higher alkyl compounds", for example the n-hexyl and n-heptyl compounds, containing a hexamethylene bridge, in comparison with the activities of the corresponding "lower alkyl compounds", represented by the n-butyl compound. These data indicate that the said n-hexyl compound is 10 times more active as a bacteriostatic agent, and 25 times more active as a bactericidal agent, than said n-butyl compound against <u>S. aureus</u>.

A paper by Cutler et al., Soap and Chemical Specialties, 1966(2), 45-49 and 101-103, is concerned with compounds of the formula V and other bisbiguanides. On pages 48 and 49 of that paper it is stated that, although previous workers (Davies et al., ibid.) noted "the need for the terminal aromatic nucleus" in bisbiguanides of the type in question, Cutler et al.'s hypothesis, which is discussed in their paper, predicted that a bisbiguanide in which the aryl radical was replaced by an alkyl radical of the proper chain length also should have high antimicrobial activity. It is stated that this has actually been found to be the case; in preliminary tests the following three compounds stood out as highly active antibacterial agents:-

$$R''NH.C(:NH)NH.C(:NH)NH-(CH_2)_6-NH.C(:NH)NH.C(:NH)NHR'' \qquad VII$$

wherein R" stands for a hexyl (presumably n-hexyl), heptyl (presumably n-heptyl) or 2-ethylhexyl radical. It is further stated that the n-butyl homologue of these compounds, which was described by Rose and Swain, ibid., was considerably less active, being especially so in the case of *Pseudomonas aeruginosa*, against which it was only 1/25th as active as the most potent member of the series.

The present invention is based upon the discovery that certain novel bisbiguanides in which the end groups are lower alkyl (or related) radicals, surprisingly have very good antibacterial properties. This is particularly unexpected in view of the prior art, discussed above, which teaches quite clearly that

known bisbiguanides with lower alkyl end groups are very much less effective as bacteriostatic or bacteriocidal compounds than the corresponding hexyl, heptyl and 2-ethylhexyl derivatives. In the compounds of the present invention, we have found, surprisingly, that this is not, in fact, the case.

Thus according to the present invention there are provided bisbiguanides of the formula:-

$$R^1R^2N.C(:NR^5)NH.C(:NH)NH\text{-}X\text{-}NH.C(:NH)NH.C(:NR^6)NR^3R^4 \quad \text{VIII}$$

or a tautomeric form thereof, wherein each of $R^1$, $R^2$, $R^3$, and $R^4$, which may be the same or different, is hydrogen, an alkyl radical of up to 5 carbon atoms (1-5C) such that $R^1$, $R^2$, $R^3$ and $R^4$ together contain 4 to 12 carbon atoms, or a 3-5C alkenyl radical; or $R^1$, $R^2$ and the nitrogen atom to which they are attached, or $R^3$ and $R^4$ and the nitrogen atom to which they are attached, which may be the same or different, are each a 1-azetidinyl, 1-pyrrolidinyl, piperidino, hexamethyleneimino, heptamethyleneimino, morpholino or 4-(1-8C alkanoyl)-1-piperazinyl radical, each of which may bear 1-3C alkyl substituents; provided that at least one of $R^1$, $R^2$, $R^3$, and $R^4$ is other than hydrogen; $R^5$ and $R^6$, which may be the same or different, are each hydrogen or a 1-8C alkyl radical; and X is a 7-20C linking group which is a straight chain alkylene radical, or a straight chain alkylene radical bearing one or more 1-3C alkyl substituents, of which any pair or pairs which are attached to different carbon atoms may be joined so as to form, together with the intervening carbon atom or atoms of the alkylene radical, a cycloalkyl radical or radicals; and the acid addition salts thereof.

Each of $R^1$, $R^2$, $R^3$ and $R^4$ may be, for example, a methyl, ethyl, n-propyl, isopropyl, n-butyl, sec-butyl, isobutyl, n-pentyl, neopentyl or allyl radical, and such radicals of 3 or 4 carbon atoms are preferred.

When $R^1$ and $R^2$, and $R^3$ and $R^4$, together with the nitrogen atom to which they are attached, form a heterocyclic radical, preferred such radicals are the 1-pyrrolidinyl and piperidino radicals, and a preferred alkyl-substituted such radical is, for example, the 2,6-dimethylmorpholino radical.

Each of $R^5$ and $R^6$ may be, for example, a methyl, ethyl, n-propyl, isopropyl, n-butyl, sec-butyl, isobutyl, n-pentyl, neo-pentyl, n-hexyl, n-heptyl, n-octyl or 2-ethylhexyl radical.

The linking group X may be, for example, a heptamethylene, octamethylene, nonamethylene, decamethylene, undecamethylene, dodecamethylene or hexadecamethylene radical. Suitable alkyl substituents on a straight chain alkylene radical X, are, for example, methyl, ethyl and n-propyl radicals, and a suitable value for X in which two pairs of such alkyl radicals are joined together is, for example, the methylenebis(4-cyclohexyl) diradical.

Preferred values for X are the undecamethylene, dodecamethylene and methylenebis(4-cyclohexyl) radicals.

A preferred group of compounds of the invention comprises those wherein the groups $R^1$, $R^2$, $R^3$, $R^4$ and X together contain 16 to 28, preferably 18 to 24, carbon atoms and $R^5$ and $R^6$ are hydrogen. Particularly preferred, because of their

high antibacterial activity, are those compounds wherein $R^1$, $R^2$, $R^3$ and $R^4$ are all alkyl radicals.

Particular preferred compounds of the invention are 1,12-dodecanebis(5-n-butylbiguanide), 1,12-dodecanebis(5-isobutylbiguanide), 1,12-dodecane-bis(5-n-pentylbiguanide) and 1,12-dodecanebis(5-neopentylbiguanide), and their dihydrochlorides.

The acid-addition salts of the invention may be derived from an inorganic or organic acid. In most circumstances it is preferable that the salts be derived from an acid which affords an anion which is suitable for human usage, for example a pharmaceutically acceptable anion. Examples of such acids are hydrochloric, hydrobromic, phosphoric, sulphuric, D-gluconic, 2-pyrrolidone-5- carboxylic, methanesulphonic, carbonic, lactic, acetic and glutamic acids.

A preferred group of bisbiguanide derivatives of the invention comprises compounds of the formula VIII wherein $R^1$ and $R^3$ are each 1-5C alkyl, $R^2$, $R^4$, $R^5$ and $R^6$ are each hydrogen, and X is undecamethylene or dodecamethylene, and within this group, the compounds wherein $R^1$ and $R^3$ are each n-butyl, sec- butyl, isobutyl, n-pentyl or neopentyl are especially preferred.

According to a further feature of the invention there is provided a process for the manufacture of the compounds of the invention wherein $R^5$ and $R^6$ are both hydrogen, which comprises reacting a bis-cyanoguanidine of the formula

NC.NH.C(:NH)NH-X-NH.C(:NH).NH.CN X

with an amine, $R^1R^2NH$, or with two different amines, $R^1R^2NH$ and $R^3R^4NH$, in the form of an acid-addition salt thereof, wherein X, $R^1$, $R^2$, $R^3$ and $R^4$ have the meanings stated above, at a temperature of 100° to 170°C.

A suitable amine salt is, for example, the hydrochloride. The reactants are heated together until the reaction is complete. The reaction proceeds fastest at the higher temperatures, but if thermal stability at higher temperatures appears to be a problem, the reaction can be carried out at lower temperature for a longer period. The reactants are most conveniently melted together in the absence of a solvent, but if desired an inert solvent such as 2-methoxyethanol, 2-ethoxyethanol, nitrobenzene, sulpholane, isopropanol, n-butanol, ethylene glycol dimethyl ether or water, or a mixture of such solvents may be used.

The bis-cyanoguanidine starting material of the formula X, used in the above process, may be obtained by reacting the appropriate diamine $NH_2$-X-$NH_2$, in the form of an acid addition salt, conveniently the dihydrochloride, with sodium dicyanamide, in conventional manner.

According to a further feature of the invention there is provided a process for the manufacture of the compounds of the invention which comprises reacting a diamine of the formula $H_2N$-X-$NH_2$, in the form of an acid-addition salt, with a cyanoguanidine of the formula:-

$R^1R^2N.C(:NR^5)NH.CN$ XI

or with a cyanoguanidine of the formula XI and a cyanoguanidine of the formula:-

$R^3R^4N.C(:NR^6)NH.CN$ XII

and wherein X, $R^1$, $R^2$, $R^3$, $R^4$, $R^5$ and $R^6$ have the meanings stated above, at a temperature of 100° to 170°C.

A suitable salt of the diamine is, for example, the dihydrochloride. The reactants are heated together until the reaction is complete. The reaction proceeds fastest at the higher temperatures, but if thermal stability is a problem, the reaction temperature should be lowered, and the reaction allowed to proceed for longer. If a melt can be formed at those temperatures the reactants are conveniently melted together in the absence of a solvent. If not, or alternatively, the reactants are heated together in a suitable inert solvent, for example those mentioned above.

The cyanoguanidine starting materials of the formula XI and XII, wherein $R^5$ and $R^6$ are hydrogen, used in the above process, may be obtained in conventional manner by reacting an amine $R^1R^2NH$ or $R^3R^4NH$, in the form of an acid-addition salt, conveniently the dihydrochloride, with sodium dicyanamide.

The cyanoguanidines of the formulae XI and XII wherein $R^5$ and $R^6$ are other than hydrogen, which may be used as starting materials in the above

process, may be obtained by reacting a dialkyl (cyano-imido)dithio-carbonate, for example dimethyl (cyano-imido)dithio-carbonate, $(MeS)_2C:N.CN$, with appropriate amines $R^1R^2NH$ and $R^5NH_2$ (which are preferably the same), or $R^3R^4NH$ and $R^6NH_2$.

The acid-addition salts of the invention are obtained by conventional means.

The antibacterial activity of the compounds of the invention has been measured by the well-known minimum inhibitory concentration (MIC) test. Neat or diluted broth cultures of eight Gram positive organisms (*Streptococcus pyogenes*, *S. faecalis*, 3 strains of *Staphyloccus aureus*, *Listeria monocytogenes*, *Streptococcus mutans*, *S. sanguis*), *Candida albicans* and fourteen Gram negative organisms (4 strains of *Escherichia coli*, *Salmonella dublin*, *Klebsiella aerogenes*, *K. pneumoniae*, *E. cloacae*, *Serratia marcescens*, *Proteus vulgaris*, *P. mirabilis* and 3 strains of *Pseudomonas aeruginosa*) were inoculated by means of an automatic microtitre inoculator on the surface of nutrient agar plates containing two-fold or five-fold dilutions of a test compound. After incubation over-night at 37°C., the MIC's of the test compound are read. The geometric mean MIC's for the eight Gram positive organisms plus *Candida*, and 14 Gram negative organisms are then calculated for each test compound.

Depending upon its precise chemical structure, a compound of the invention has a mean MIC within the range 1-12 µg./ml. in agar against the 8 Gram positive organisms and *Candida*, and 20-250 µg./ml. in agar against the 14 Gram negative organisms.

The preferred compounds of the invention have an acute $LD_{50}$ within the accepted limits for compounds used topically, are of low irritancy in the Draize test on intact rabbit skin, are negative in the Ames test for mutagenicity, and are non-sensitizing in the Magnusson and Kligman contact sensitivity test in guinea-pigs.

Because of their antibacterial properties, the compounds of the invention are useful for many purposes, for example:-

(a) in medical and veterinary practice for the disinfection of wounds, membranes and/or skin tissue;

(b) for the sterilisation of surgical instruments and other medical apparatus and equipment, for example respirators, ventilators, incubators, humidifiers, etc.;

(c) for incorporation in toothpastes and mouthwashes for inhibiting the formation of dental plaque, and gingivitis;

(d) for the disinfection of hard surfaces, for example plant and equipment used in the food and drink industries, and floors and walls in the home, factories and hospitals;

(e) for the disinfection of textiles, for example blankets, overalls, bed-linen etc.;

(f) for the control of microbiological slime in the pulp and paper industries;

(g) for the control of micro-organisms in swimming pools, cooling water, pasteuriser water, aqueous oil emulsions such as metal working fluids, and other circulating water systems; and

(h) as plant bactericides and fungicides.

Compounds of the invention also possess useful antifungal activity against, for example, _Candida albicans_ and _Trichophyton mentagrophytes_, and algicidal and anti-yeast properties.

According to a further feature of the invention there are provided antibacterial or antifungal compositions comprising a compound of the formula VIII wherein $R^1$, $R^2$, $R^3$, $R^4$, $R^5$, $R^6$ and X have the meanings stated above, or an acid-addition salt thereof, and an inert diluent or carrier therefor.

The antibacterial or antifungal compositions of the invention are prepared by conventional means using conventional excipients. They include, for example, aqueous solutions, for example concentrated aqueous solutions, sterile, ready-to-use aqueous solutions, aqueous dispersions, and emulsions, for example oil-in-water emulsions, for example aqueous gels, creams, ointments and pastes. Suitable excipients include, for example, wetting agents, dispersing agents, emulsifying agents, gelling agents or thickening agents.

According to a further feature of the invention, there is provided a contraceptive method which comprises applying to sperm, or the locus of sperm, a spermicidal, sperm-immobilising or mucospissic amount of a compound of the invention of the formula VIII.

In one aspect of this method, the compound of the formula VII, when applied to vaginal mucus at a

suitable concentration, very rapidly increases its viscosity, to the extent that it becomes essentially impenetrable to sperm, and forms a physical barrier to conception in the same way as a rubber sheath or a diaphragm cap.

Besides increasing the viscosity of vaginal mucus, when the mucus comes into contact with a bisbiguanide compound of the formula VII, other changes occur in its intrinsic properties, such as its morphology, rheology and water uptake and visco-elastic properties, which can also effect its penetrability to sperm. The compounds also possess spermicidal or sperm-immobilising properties.

In vitro, the compounds of the formula VII exert a useful contraceptive effect at concentrations down to about $10^{-3}$ to $10^{-4}$%, and a suitable amount to be applied to the human vagina for contraceptive purposes is from 1.0 g. to $10^{-4}$g.

The compound of the formula VIII may be applied to the vagina in conventional manner, for example as a pessary, cream, liquid douche, gel, aerosol foam or impregnated tampon, or in a controlled delivery device of the compound in a polymer matrix.

According to a further feature of the invention there is provided a compound of the formula VIII, or a composition thereof, for use as a contraceptive.

The invention is illustrated but not limited by the following Examples in which the temperatures are expressed in degrees Celsius:-

Example 1

A mixture of isopropylamine hydrochloride (2.9g.) and 1,12-dodecanebis(3-cyanoguanidine) (5.1g.) was stirred and heated at 140-150° in an oil bath for 3 hr. The clear melt was allowed to cool to ambient temperature, and then crystallised from methanol/acetone. The crystallisation was repeated twice more, and there was thus obtained 1,12-dodecanebis(5-isopropylbiguanide) dihydrochloride, m.p. 210-211°.

The cyanoguanidine derivative used as starting material was obtained as follows:-

A mixture of sodium dicyanamide (72g., 95% w/w strength) and 1,12-dodecanediamine dihydrochloride (100g.) in n-butanol (500ml.) was stirred and heated under reflux (approx. 120°) for 4 hr. The mixture was filtered whilst hot, and the filtrate was evaporated under reduced pressure. The residual solid was crystallised from aqueous acetone to give 1,12-dodecanebis(3-cyanoguanidine), m.p. 200-204°.

Example 2

A mixture of 1,11-undecanediamine dihydrochloride (6.0g.) and 1-neopentyl-3-cyanoguanidine (7.1g.) was stirred and heated at 140-150° in an oil bath for 3 hrs. The clear colourless melt, which formed initially, solidified after two hours. The solid was dissolved in the minimum quantity of refluxing ethanol and acetone was added under reflux until the solution became cloudy. The mixture was allowed to cool to ambient temperature, during which time a white crystalline solid separated out. The mixture was filtered and the solid residue dried in a vacuum oven under reduced pressure and at room

temperature for 24 hours. There was thus obtained 1,11-undecanebis(5-neopentyl-biguanide) dihydrochloride, m.p. 207-210°.

The cyanoguanidine derivative used as starting material was obtained as follows:-

A mixture of neopentylamine hydrochloride (62g.) and sodium dicyanamide (50g., 95% w/w strength) in n-butanol (500ml.) was stirred and heated under reflux (approx. 120°) for 4 hr. The mixture was allowed to cool to ambient temperature, and then filtered, and the filtrate was evaporated under reduced pressure. The residual solid was crystallised from water to give 1-neopentyl-3-cyanoguanidine, m.p. 177-188°.

Examples 3-19

The compounds listed in the following table were made, as the dihydrochlorides, by the process described in Example 1, using an equivalent amount of the amine hydrochloride $R^1R^2NH.HCl$, or a mixture of amines $R^1R^2NH.HCl$ and $R^3R^4NH.HCl$, in place of isopropylamine hydrochloride, and an equivalent amount of the appropriate bis-cyanoguanidine derivative in place of the 1,12-dodecanebis(3-cyanoguanidine):-

$$R^1R^2N.C(:NH)NH.C(:NH)NH\text{-}(CH_2)_n\text{-}NH.C(:NH)NH.C(:NH)NR^3R^4.2HCl \qquad XII$$

| Ex.No. | $R^1$ | $R^2$ | $R^3$ | $R^4$ | n | M.p. |
|---|---|---|---|---|---|---|
| 3 | pentyl | H | pentyl | H | 8 | 209-210 |
| 4 | neopentyl | H | neopentyl | H | 8 | 240-243 |
| 5 | neopentyl | H | neopentyl | H | 10 | 218-230 |
| 6 | isopropyl | H | isopropyl | H | 11 | 198-202 |
| 7 | propyl | H | propyl | H | 11 | 145-149 |
| 8 | isobutyl | H | isobutyl | H | 11 | 178-183 |
| 9 | ethyl | H | ethyl | H | 12 | 120-126 |
| 10 | propyl | H | propyl | H | 12 | 144-145 |
| 11 | butyl | H | butyl | H | 12 | 126-143 |
| 12 | sec-butyl | H | sec-butyl | H | 12 | 210-212 |
| 13 | methyl | ethyl | methyl | ethyl | 12 | 165-168 |
| 14 | ethyl | ethyl | ethyl | ethyl | 12 | 167-171 |
| 15 | propyl | propyl | propyl | propyl | 12 | 159-162 |
| 16 | $-(CH_2)_5-$ | | $-(CH_2)_5-$ | | 12 | 160-165 |
| 17 | $-(CH_2)_6-$ | | $-(CH_2)_6-$ | | 12 | 149-151 |
| 18 | ethyl | propyl | ethyl | propyl | 12 | 182 |
| 19 | methyl | butyl | methyl | butyl | 12 | 197-200 |

Examples 20-28

The following compounds of the formula XII (see Examples 3-19) were made, as the dihydrochlorides, by the process described in Example 2, using an equivalent amount of the diamine dihydrochloride $H_2N$-X-$NH_2$.2HCl in place of 1,11-undecanediamine dihydrochloride, and an equivalent amount of the appropriate cyanoguanidine derivative in place of 1-neopentyl-3-cyanoguanidine:-

| Ex.No. | $R^1$ | $R^2$ | $R^3$ | $R^4$ | X | M.p. |
|---|---|---|---|---|---|---|
| 20 | neopentyl | H | neopentyl | H | $(CH_2)_7$ | 235-240 |
| 21 | neopentyl | H | neopentyl | H | $(CH_2)_9$ | 150-170 |
| 22 | isobutyl | H | isobutyl | H | $(CH_2)_{12}$ | 205-208 |
| 23 | pentyl | H | pentyl | H | $(CH_2)_{12}$ | 156-164 |
| 24 | neopentyl | H | neopentyl | H | $(CH_2)_{12}$ | 204-206 |
| 25 | propyl | H | propyl | H | 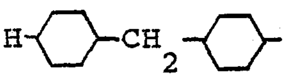 | 280-283 |
| 26 | butyl | H | butyl | H | " | 250-255 |
| 27 | isobutyl | H | isobutyl | H | " | 220-227 |
| 28 | isopropyl | iso-propyl | isopropyl | iso-propyl | $(CH_2)_{12}$ | 160-166 |

The 3-cyano-1,1-di-isopropylguanidine used as starting material for Example 28 was obtained by reacting sodium dicyanamide with di-isopropylamine dihydrochloride m.p. 121-123°, crystallised from aqueous ethanol.

Example 30

A mixture of 1-methyl-3-cyanoguanidine (1.25g.) and 1,16-hexadecanediamine dihydrochloride (2.0g.) was heated, with stirring in an oil bath at 140° for 3hr. The melt was allowed to cool to ambient temperature, and it crystallised after three days. The solid was triturated with warm acetone (40ml.) and then filtered, to give 1,16-hexadecane-bis(5-methylbiguanide), m.p. 78-79.5°.

Example 31

A mixture of 1-ethyl-3-cyanoguanidine (1.7g.) and 1,16-hexadecanediamine dihydrochloride (2.0g.) was heated with stirring in an oil bath at 140° for 3 hr. The melt was allowed to cool to ambient temperature, and was then dissolved in the minimum volume of hot ethanol. Acetone was then added until a solid just began to precipitate. The mixture was then kept at ambient temperature until precipitation was complete. The mixture was filtered to give 1,16-hexadecanebis(5-ethylbiguanide), m.p. 129-130.5°.

Example 32

In analogous manner to that described in Example 31, 1-isopropyl-3-cyanoguanidine and 1,16-hexadecanediamine dihydrochloride were reacted together to give 1,16-hexadecanebis(5-isopropylbiguanide), m.p. 209-210.5°.

Example 33

Ethylamine hydrochloride (0.73g.), neopentylamine hydrochloride (1.2g.) and 1,12-dodecanebis(3-cyanoguanidine) (2.5g.) were ground together, and the mixture was heated in an oil bath at 145° for 3 hr. The melt was allowed to cool to ambient temperature, and then dissolved in the minimum volume of methanol/water (1:1 v/v). The product was purified by reverse phase high pressure liquid chromatography on a C-18 reverse phase silica column (0.04mm-0.63mm particle size, 30 x 3.8cm.; flow-rate 25ml./min (at 100 psi); solvent as above; detector - U.V. set at 230nm., absorbance at 2. The fractions containing the desired product were identified by thin layer chromatography,

combined and evaporated to dryness under reduced pressure. The resulting gum was triturated with acetone and the mixture filtered to give, as the solid residue, 12-(5-ethylbiguanido)-dodecyl(5-neopentyl-neopentylbiguanide), m.p. 176.5-179°.

Example 34

A mixture of N-methylhexylamine hydrochloride (0.95g.) and 1,6-hexanebis(3-cyanoguanidine) (0.78g.) was fused at 145° for 3 hours. The reaction mixture was purified by medium pressure liquid chromatography (MPLC), eluting with aqueous methanol, and the fractions containing bisbiguanide (U.V. absorption at 260nm.) were combined and evaporated to dryness to give 1,6-hexanebis(5-hexyl-5-methylbiguanide) dihydrochloride, m.p. 185-186°.

Example 35

The process described in Example 34 was repeated, using N-methyloctylamine in place of N-methylhexylamine, to give 1,6-hexanebis(5-methyl-5-octylbiguanide) dihydrochloride, m.p. 184-185°.

Example 36

A mixture of 1,6-hexanediamine dihydrochloride (0.78g.) and 3-cyano-1-isobutyl-2-isopropylguanidine (1.5g.) was fused at 160° for 3.5 hours. The reaction mixture was purified by MPLC, eluting with aqueous methanol, and the fractions containing bisbiguanide (U.V. absorption at 260 nm.) were combined and evaporated to dryness to give 1,6-hexanebis(4-isobutyl-5-isopropylbiguanide) dihydrochloride, m.p. 269-271°.

The 3-cyano-1-isobutyl-2-isopropylguanidine used as starting material in the above process was manufactured as follows:-

To a stirred solution of isobutylamine (10g.) in ethanol (400ml.) was added a solution of dimethyl (cyanoimido)dithiocarbonate (9.07g.) in ethanol (200ml.) and the mixture was stirred overnight at room temperature. The solvent was evaporated under reduced pressure to give 3-cyano-1-isobutyl-2-methylisothiourea as a colourless solid, m.p. 118°.

This material (2g.) without further purification, isopropylamine (5ml.) and dimethylsulphoxide (20ml.) were heated in a sealed tube at 100° for 72 hours. The tube was cooled and opened, and the solvent was removed by evaporation under reduced pressure to give a solid which was recrystallised from aqueous methanol to give 3-cyano-1-isobutyl-2-isopropylguanidine, m.p. 135-136°.

Examples 37-46

The process described in Example 36 was repeated, using the appropriate diamine and substituted cyanoguanidine starting materials, to manufacture the following compounds of the formula VIII:-

| Ex. | $R^1(=R^3)$ | $R^2(=R^4)$ | $R^5(=R^6)$ | X | Reaction Temp. | Time. | M.p. |
|---|---|---|---|---|---|---|---|
| 37 | isobutyl | H | isobutyl | $(CH_2)_6$ | 160 | 3.5 | 242-245 |
| 38 | isopentyl | H | isobutyl | $(CH_2)_6$ | 160 | 3 | 253 |
| 39 | methyl | H | isopentyl | $(CH_2)_6$ | 160 | 2 | 95 |
| 40 | ethyl | H | isopentyl | $(CH_2)_6$ | 160 | 2 | 189-191 |
| 41 | isopropyl | H | isopentyl | $(CH_2)_6$ | 160 | 2 | 245-246 |
| 42 | isopentyl | iso-pentyl | H | $(CH_2)_6$ | 145 | 3 | 147-151 |
| 43 | ethyl | butyl | H | $(CH_2)_6$ | 145 | 3 | 179-181 |
| 44 | isobutyl | iso-butyl | H | $(CH_2)_{12}$ | 145 | 3 | 144-147 |
| 45 | ethyl | H | ethyl | $(CH_2)_{12}$ | 145 | 3 | 190-194 |
| 46 | ethyl | methyl | H | $(CH_2)_{12}$ | 145 | 3 | 165-168 |

The 1,2-disubstituted-3-cyanoguanidines used as starting materials in the above process were manufactured by the process described in the second part of Example 36, using appropriate amines.

$R^1NH.C(:NR^5).NH.CN$

| Ex. | $R^1$ | $R^5$ | Crystallisation solvent | M.p. |
|---|---|---|---|---|
| 37 | isobutyl | isobutyl | aqueous ethanol | 80-82 |
| 38 | isopentyl | isobutyl | a | 79-81 |
| 39 | methyl | isopentyl | petroleum ether | 68-70 |
| 40 | ethyl | isopentyl | " | 69-71 |
| 41 | isopropyl | isopentyl | " | 100-101 |
| 45 | ethyl | ethyl | " | 110-113 |

a - triturated with petroleum ether.

Example 47

The process described in Example 36 was repeated, using 1,12-dodecanediamine and 3-cyano-1,1,2,2-tetramethylguanidine as starting materials to give 1,12-dodecanebis(4,4,5,5-tetramethylbiguanide)dihydrochloride, as a gum.

Example 48

A mixture of 3-cyano-1,1-di-isobutylguanidine (2.45g.), 3-cyano-1-methylguanidine (0.98g.) and 1,12-dodecanediamine dihydrochloride (2.73g.) were fused together at 145° for 3 hours. The reaction product having $R_f$ = 0.5 on thin layer chromatography on silica gel, developed with a mixture of butanol : acetic acid : water (65:15:25 by volume) was separated, and purified by MPLC to give 12-(5-methylbiguanido)-dodecyl(5,5-di-isobutylbiguanide) dihydrochloride, m.p. 120-122°, crystallised from aqueous methanol.

What we claim is:-

1. A bisbiguanide of the formula:-

$$R^1R^1N.C(:NR^5)NH.C(:NH)NH\text{-}X\text{-}NH.C(:NH)NH.C(:NR^6)NR^3R^4 \qquad VIII$$

or a tautomeric form thereof, wherein each of $R^1$, $R^2$, $R^3$, and $R^4$, which may be the same or different, is hydrogen, an alkyl radical of up to 5 carbon atoms (1-5C) such that $R^1$, $R^2$, $R^3$ and $R^4$ together contain 4 to 12 carbon atoms, or a 3-5C alkenyl radical; or $R^1$, $R^2$ and the nitrogen atom to which they are attached, or $R^3$ and $R^4$ and the nitrogen atom to which they are attached, which may be the same or different, are each a 1-azetidinyl, 1-pyrrolidinyl, piperidino, hexamethyleneimino, heptamethyleneimino, morpholino or 4-(1-8C alkanoyl)-1-piperazinyl radical, each of which may bear 1-3C alkyl substituents; provided that at least one of $R^1$, $R^2$, $R^3$, and $R^4$ is other than hydrogen; $R^5$ and $R^6$ are each hydrogen or a 1-8C alkyl radical; and X is a 7-20C linking group which is a straight chain alkylene radical, or a straight chain alkylene radical bearing one or more 1-3C alkyl substituents, of which any pair or pairs which are attached to different carbon atoms may be joined so as to form, together with the intervening carbon atom or atoms of the alkylene radical, a cycloalkyl radical or radicals; and the acid addition salts thereof.

2. A bisbiguanide as claimed in claim 1 wherein each of $R^1$, $R^2$, $R^3$ and $R^4$ which may be the same or different, is a methyl, ethyl, n-propyl, isopropyl, n-butyl, sec-butyl, isobutyl, n-pentyl, neopentyl or allyl radical, or $R^1$ and $R^2$, or $R^3$ and $R^4$, together with the nitrogen atom to which they are attached, are

1-pyrrolidinyl, piperidino or 2,6-dimethylmorpholino radicals, $R^5$ and $R^6$,which may be the same or different, are each hydrogen or a methyl, ethyl, n-propyl, isopropyl, n-butyl, sec-butyl, isobutyl, n-pentyl, neo-pentyl, n-hexyl, n-heptyl, n-octyl or 2-ethylhexyl radical, X is a heptamethylene, octamethylene, nonamethylene, decamethylene, undecamethylene, dodecamethylene or hexadecamethylene radical optionally substituted by methyl, ethyl or n-propyl radicals, or X is a methylenebis(4-cyclohexyl)radical.

3. A bisbiguanide as claimed in claim 2 wherein $R^1$, $R^2$, $R^3$, $R^4$ and X together contain 16 to 28 carbon atoms, and $R^5$ and $R^6$ are hydrogen.

4. A bisbiguanide as claimed in claim 3 wherein $R^1$, $R^2$, $R^3$ and $R^4$ are all alkyl radicals, and $R^1$, $R^2$, $R^3$, $R^4$ and X together contain 18 to 24 carbon atoms.

5. The bisbiguanide compound as claimed in claim 1 which is 1,12-dodecanebis(5-n-butylbiguanide), 1,12-dodecanebis(5-isobutylbiguanide), 1,12-dodecane-bis-(5-n-pentylbiguanide) or 1,12-dodecanebis(5-neopentyl-biguanide), or dihydrochloride thereof.

6. A bisbiguanide as claimed in claim 1 which is in the form of a salt with hydrochloric, hydrobromic, phosphoric, sulphuric, D-gluconic, 2-pyrrolidone-5-carboxylic, methanesulphonic, carbonic, lactic, acetic or glutamic acid.

7. A bisbiguanide as claimed in claim 1 wherein $R^1$ and $R^3$ are ech a 1-5C alkyl radical, $R^2$, $R^4$, $R^5$ and $R^6$ are each hydrogen, and X is an undecamethylene or dodecamethylene radical.

8. A bisbiguanide as claimed in claim 7 wherein $R^1$ and $R^3$ are each n-butyl, sec-butyl, isobutyl, n-pentyl or neopentyl.

9. A process for the manufacture of a bisbiguanide as claimed in claim 1 which comprises:

a) for those compounds wherein $R^5$ and $R^6$ are both hydrogen, reacting a bis-cyanoguanidine of the formula:-

NC.NH.C(:NH)NH-X-NH.C(:NH).NH.CN X

with an amine, $R^1R^2NH$ or with two different amines, $R^1R^2NH$ and $R^3R^4NH$, in the form of an acid-addition salt thereof, wherein X, $R^1$, $R^2$, $R^3$ and $R^4$ have the meanings stated in claim 1, at a temperature of 100° to 170°C; or

b) reacting a diamine of the formula $H_2N-X-NH_2$, in the form of an acid-addition salt, with a cyanoguanidine of the formula:-

$R^1R^2N.C(:NR^5)NH.CN$ XI

or with a cyanoguanidine of the formula XI and a cyanoguanidine of the formula:-

$R^3R^4N.C(:NR^6)NH.CN$ XII

and wherein X, $R^1$, $R^2$, $R^3$, $R^4$, $R^5$ and $R^6$ have the meanings stated in claim 1, at a temperature of 100° to 170°C.

10. An antibacterial or antifungal composition comprising a bisbiguanide as claimed in claim 1 and an inert diluent or crrier therefor.

11. A method of obtaining an antibacterial or antifungal, effect:

a) in medical and veterinary practice for the disinfection of wounds, membranes or skin tissue;

b) in the sterilisation of surgical instructions and other medical apparatus and equipment;

c) in toothpastes and mouthwashes for inhibiting gingivitis and the formtion of dental plaque;

d) in the disinfection of hard surfaces;

e) in the disinfection of textiles;

f) in the control of microbiological shine in the pulp and paper industries;

g) in the control of micro-organisms in swimming pools, cooling water, pasteuriser water, aqueous oil emulsions and other circulating water systems; or

h) against plant bacterial and/or fungi; which comprises applying an antibacterially-effective or antifunglly-effective amount of a bisbiguanide as claimed in claim 1 to the bacterially affected locus.

12. A contraceptive method which comprises applying to sperm, or the locus of sperm, a spermicidal, sperm-immobilising or mucospissic amount of a compound as claimed in claim 1.

DS32712
SC05
23 Mar 84

JOHN DA... ...KINSON

AUTHORISED REPRESENTATIVE
General Authorisation No. 37

0126558

European Patent Office

# EUROPEAN SEARCH REPORT

Application number

EP 84 30 2817

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,X | GB-A-1 095 902 (STERLING DRUG) * Claims; examples * ----- | 1-12 | C 07 C 129/16<br>C 07 D 295/20<br>A 01 N 47/44<br>A 61 K 7/22 |
| | | | **TECHNICAL FIELDS SEARCHED (Int. Cl. 3)** |
| | | | C 07 C 129/00 |

The present search report has been drawn up for all claims

| Place of search | Date of completion of the search | Examiner |
|---|---|---|
| THE HAGUE | 17-08-1984 | GAUTIER R.H.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82